# EUROPEAN PATENT APPLICATION

(11) **EP 4 119 671 A1**
(43) Date of publication of application: **18.01.2023**
(21) Application number: 21768682.3
(22) Date of filing: 08.03.2021
(51) Int. Cl.: C12P 17/16, C07D 401/04

(54) **METHOD FOR SYNTHESIZING (S)-NICOTINE**

(30) Priority: 10.03.2020 CN 202010172401
(71) Applicant: Porton Pharma Solutions Ltd., Chongqing 401220 (CN)
(72) Inventor: LIN, Wenqing, Chongqing 401120 (CN); ZHENG, Hongjie, Chongqing 401120 (CN); CHEN, Zegcong, Chongqing 401120 (CN); ZHU, Jianping, Chongqing 401120 (CN); YUE, Yongtang, Chongqing 401120 (CN); HU, Jicheng, Chongqing 401120 (CN); WANG, Jianchong, Chongqing 401120 (CN); LI, Lingyu, Chongqing 401120 (CN); LIU, Xiaobo, Chongqing 401120 (CN)
(74) Representative: Regimbeau
(86) International application number: PCT/CN2021/079492
(87) International publication number: WO 2021/180019

(57) **Abstract**

Provided is a method for synthesizing (S)-nicotine, comprising: under the condition of a coenzyme circulation system, by using a coenzyme as a hydrogen donor, and by using imine reductase as a catalyst, catalyzing and reducing myosmine to (S)-nornicotine, and then subjecting (S)-nornicotine to a methylation reaction so as to obtain (S)-nicotine.

## Description

### TECHNICAL FIELD

The present application belongs to the technical field of organic synthesis and, in particular, relates to a method for synthesizing (*S*)-nicotine, especially a method for synthesizing (*S*)-nicotine with a high chemical purity and a high optical purity.

### BACKGROUND

(*S*)-nicotine (nicotine) is an alkaloid present in solanaceous plants (Solanum) and an important component of tobacco. Tobacco leaves contain 1.5% to 3.5% of (*S*)-nicotine. At present, the most main method for acquiring nicotine is to extract nicotine from tobacco leaves. Through having been reported, a chemical synthesis method is immature and much higher than the extraction method in cost. The chemical synthesis methods having been reported so far include a chemical resolution method, an asymmetric hydrogenation method, a chiral auxiliary method, etc.

Patent US20160326134 reports a method where methyl nicotinate as a raw material is condensed with NMP and subjected to intramolecular rearrangement, reduction, and chiral resolution to obtain (*S*)-nicotine. The synthesis route of (*S*)-nicotine is shown below.

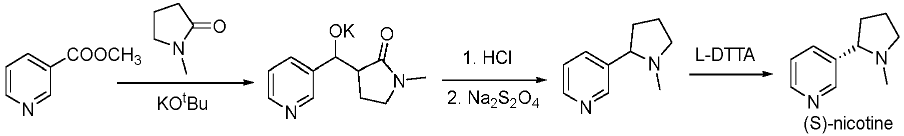

A document (Peyton Jacob, Resolution of (±)-5-Bromonornicotine. Synthesis of (R)-and (S)-Nornicotine of High Enantiomeric Purity, The Journal of Organic Chemistry 1982, 4165-4167) reports a method for obtaining (*S*)-nornicotine with a chiral acid as a resolution agent through processes including resolution, reductive debromination, etc. (*S*)-nornicotine may be further subjected to methylation to conveniently obtain (*S*)-nicotine. The synthesis route is shown below. However, the chemical resolution method has the disadvantages of a low yield and a high cost.

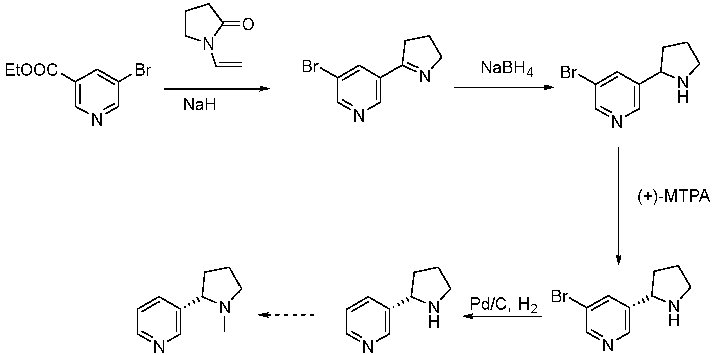

A document (Gui Guo, Dong-Wei Sun, Shuang Yang, et al, Iridium-Catalyzed Asymmetric Hydrogenation of 2-Pyridyl Cyclic Imines: A Highly Enantioselective Approach to Nicotine Derivatives, Journal of the American Chemical Society, 2015, 90-93) reports a route for synthesizing nicotine by an asymmetric catalytic hydrogenation method. The synthesis route of nicotine is shown below.

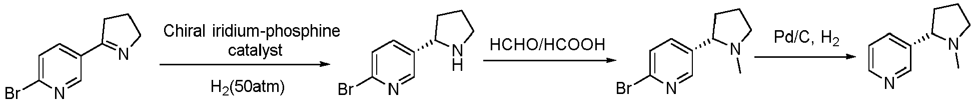

However, the asymmetric catalytic hydrogenation method requires the use of an expensive chiral metal catalyst and thus has a high cost, the reactions need to be performed under high pressure, the equipment needs a large investment, and the use of hydrogen also presents a potential safety hazard. Additionally, the document shows that only a hexa-substituted substrate is catalyzed with relatively good performance, and there is no technical breakthrough for the direct hydrogenation of myosmine.

A document (Teck-Peng Loh, Jian-Rong Zhou, Xu-Ran Li, Keng-Yeow Sim, A novel reductive aminocyclization for the syntheses of chiral pyrrolidines: stereoselective syntheses of (S)-nornicotine and 2-(2'-pyrrolidl)-pyridines, Tetrahedron Letters, 1999, 7847-7850) reports a method for synthesizing (*S*)-nornicotine with galactosamine as a chiral auxiliary. (*S*)-nornicotine can be further reacted to obtain (*S*)-nicotine. The synthesis route is shown below.

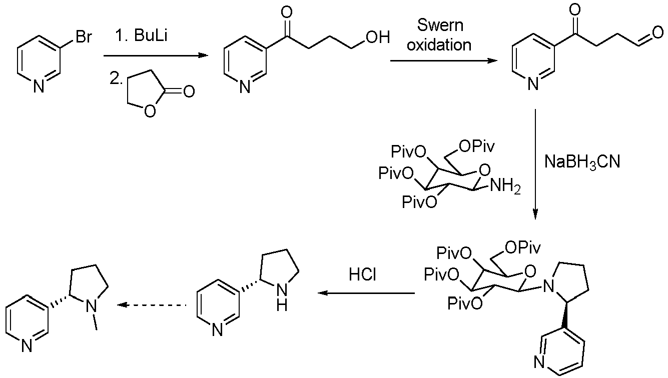

However, a stoichiometric chiral auxiliary needs to be used in this chiral auxiliary method and cannot be recycled, resulting in a high cost and no practical application value.

In summary, each chemical synthesis method for (*S*)-nicotine in the prior art has deficiencies, and there is a need to develop a more economical, effective and safe synthesis method.

### SUMMARY

In view of the deficiencies in the prior art, an object of the present application is to provide a method for synthesizing (*S*)-nicotine, especially a method for synthesizing (*S*)-nicotine with a high chemical purity and a high optical purity.

To achieve the object, the present application adopts the technical solutions described below.

The present application provides a method for synthesizing (*S*)-nicotine, including
under the condition of a coenzyme circulation system, with a coenzyme as a hydrogen donor and an imine reductase as a catalyst, subjecting myosmine to catalytic reduction to obtain (*S*)-nornicotine and subjecting (*S*)-nornicotine to methylation to obtain the (*S*)-nicotine.

The synthesis method related to the present application may be represented by the following reaction formula:

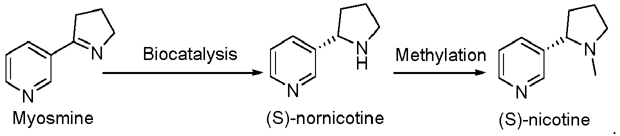

The synthesis method is simple to operate, safe and reliable, high in yield and low in cost and has very good chemical selectivity with a product purity of more than or equal to 99.5%, and also an excellent enantioselectivity with an optical purity of more than or equal to 99.5%.

The IUPAC name of myosmine related to the present application is 3-(3,4-dihydro-2H-pyrrol-5-yl)pyridine, and the IUPAC name of (*S*)-nornicotine is (*S*)-3-(pyrrolidin-2-yl)pyridine.

Preferably, the coenzyme circulation system includes a coenzyme, glucose, and a glucose dehydrogenase.

Preferably, the coenzyme includes an NADP salt and/or an NAD salt, preferably an NADP salt.

Preferably, the glucose dehydrogenase includes any one or a combination of at least two of GDH101 to GDH109. Specifically, the glucose dehydrogenase includes any one or a combination of at least two of GDH101, GDH102, GDH103, GDH104, GDH105, GDH106, GDH107, GDH108 or GDH109. GDH109 (lot number: S20191121) is preferred. The preceding glucose dehydrogenases are purchased from SyncoZymes (Shanghai) Co., Ltd.

Preferably, the imine reductase includes any one or a combination of at least two of IRED101 to IRED112, IRED1321104, IRED1321108, IRED1321110 or IRED1321114. Specifically, the imine reductase includes any one or a combination of at least two of IRED101, IRED102, IRED103, IRED104, IRED105, IRED106, IRED107, IRED108, IRED109, IRED110, IRED111, IRED112, IRED1321104, IRED1321108, IRED1321110 or IRED1321114. IRED103 (lot number: S20191121) or IRED1321110 is preferred. IRED101 to IRED112 are purchased from SyncoZymes (Shanghai) Co., Ltd., and IRED1321104, IRED1321108, IRED1321110 or IRED1321114 is independently mined from a gene bank.

Specifically, IRED1321104 is from Streptomyces Kanamyceticus, IRED1321108 is from Mesorhizobium sp. L2C084, IRED1321110 is from Salinispora pacifica, and IRED1321114 is from Mesorhizobium ciceri. The above selected imine reductase is sent to a gene synthesis company for synthesis, carry into plasmids pET28(a), and introduction into *E.coli* BL21 to construct *Escherichia coli* recombinant strain. The above recombinant strain is inoculated in an autoclaved LB medium (10 g/L sodium chloride, 10 g/L tryptone and 5 g/L yeast powder) and cultured at 37 °C and 220 RPM until OD600 is 2-3. 0.1M IPTG is added for induction at 25 °C for 16 h. After centrifugation, cells are collected, subjected to ultrasonics to be broken apart, and centrifuged to remove cell debris to obtain an enzyme solution. The enzyme solution is lyophilized to obtain the self-made enzyme powder.

Preferably, the catalytic reduction is performed at a temperature of 15-45 °C, for example, 15 °C, 20 °C, 25 °C, 30 °C, 35 °C, 40 °C, or 45 °C, etc. Other specific point values within the range may be selected, and details are not described here. 25-30 °C is preferred.

Preferably, the catalytic reduction is performed for 8-72 h, for example, 8 h, 10 h, 12 h, 16 h, 24 h, 30 h, 48 h, 56 h, 60 h, or 72 h, etc. Other specific point values within the range may be selected, and details are not described here.

Preferably, the catalytic reduction is performed under the condition of stirring at a rotational speed of 150-400 r/min, for example, 150 r/min, 200 r/min, 250 r/min, 300 r/min, 350 r/min, or 400 r/min, etc. Other specific point values within the range may be selected, and details are not described here.

Preferably, the catalytic reduction is performed in a buffer, where the buffer includes a phosphate buffer, a tris(hydroxymethyl)methylamine-hydrochloride buffer, or a triethanolamine-hydrochloride buffer.

Preferably, the buffer has a pH of 5.0-9.0, for example, pH = 5.0, pH = 5.5, pH = 6.0, pH = 6.5, pH = 7.0, pH = 7.5, pH = 8.0, pH = 8.5, or pH = 9.0, etc. Other specific point values within the range may be selected, and details are not described here. 5.8-6.5 is preferred.

Preferably, the methylation includes mixing and reacting (*S*)-nornicotine with formaldehyde and formic acid to obtain the (*S*)-nicotine.

Preferably, the methylation is performed at a temperature of 55-80 °C, for example, 55 °C, 60 °C, 65 °C, 70 °C, 75 °C, or 80 °C, etc. Other specific point values within the range may be selected, and details are not described here.

Preferably, the methylation is performed for 8-48 h, for example, 9 h, 12 h, 18 h, 26 h, 32 h, 38 h, 46 h, or 48 h, etc. Other specific point values within the range may be selected, and details are not described here.

As a preferred technical solution of the present application, the method for synthesizing (*S*)-nicotine includes mixing myosmine, a coenzyme, glucose, a glucose dehydrogenase, a buffer and an imine reductase and reacting the mixture at 15-45 °C for 8-72 h to obtain (*S*)-nornicotine, and then mixing (*S*)-nornicotine with formaldehyde and formic acid and reacting the mixture at 55-80 °C for 8-48 h to obtain the (*S*)-nicotine.

Preferably, a mass ratio of myosmine, the coenzyme, and the imine reductase is 1:(0.001-0.05):(0.05-0.1).

Preferably, a mass ratio of the coenzyme, glucose, and the glucose dehydrogenase is (0.001-0.05):(1.2-2.4):(0.01-0.05). With the above mass ratio, the preparation method has better chemical selectivity.

Compared with the prior art, the present application has the beneficial effects described below.

The method for synthesizing (*S*)-nicotine in the present application is simple to operate, safe and reliable, high in yield and low in cost and has very good chemical selectivity with a product purity of more than or equal to 99.5%, and also an excellent enantioselectivity with an optical purity of more than or equal to 99.5%.

### BRIEF DESCRIPTION OF DRAWINGS

FIG. 1 is a proton nuclear magnetic resonance (¹H NMR) characterization spectrum of a myosmine sample in Example 1;
FIG. 2 is a liquid chromatogram showing the chemical purity of a myosmine sample in Example 1;
FIG. 3 is a ¹H NMR characterization spectrum of an (*S*)-nornicotine sample in Example 2;
FIG. 4 is a liquid chromatogram showing the chemical purity of an (*S*)-nornicotine sample in Example 2;
FIG. 5 is a chiral chromatogram of a nornicotine racemate sample;
FIG. 6 is a chiral chromatogram of an (*S*)-nornicotine sample in Example 2;
FIG. 7 is a liquid chromatogram showing the chemical purity of an (*S*)-nornicotine sample in Example 3;
FIG. 8 is a chiral chromatogram of an (*S*)-nornicotine sample in Example 3;
FIG. 9 is a liquid chromatogram showing the chemical purity of an (*S*)-nornicotine sample in Example 6;
FIG. 10 is a chiral chromatogram of an (*S*)-nornicotine sample in Example 6;
FIG. 11 is a ¹H NMR characterization spectrum of an (*S*)-nicotine sample in Example 7;
FIG. 12 is a liquid chromatogram showing the chemical purity of an (*S*)-nicotine sample in Example 7;
FIG. 13 is a chiral chromatogram of a nicotine racemate sample;
FIG. 14 is a chiral chromatogram of an (*S*)-nicotine sample in Example 7;
FIG. 15 is a liquid chromatogram showing the chemical purity of an (*S*)-nicotine sample in Example 8; and
FIG. 16 is a chiral chromatogram of an (*S*)-nicotine sample in Example 8.

### DETAILED DESCRIPTION

Technical solutions of the present application are further described below through specific examples. It is to be understood by those skilled in the art that the examples are used for a better understanding of the present application and are not to be construed as specific limitations to the present application.

### Example 1

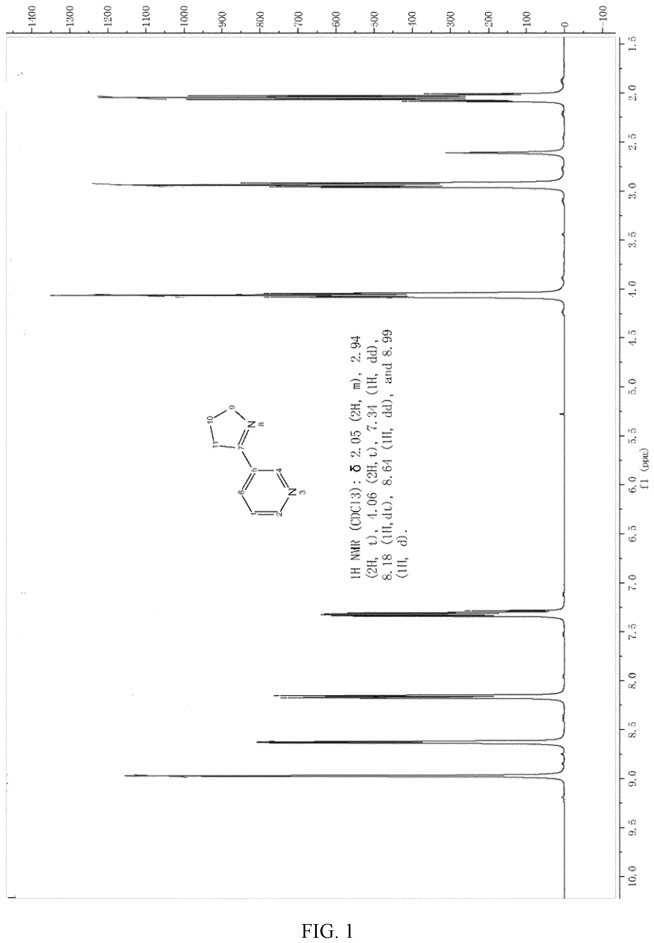

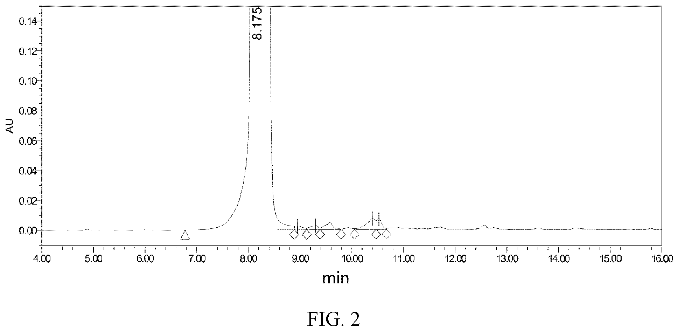

In this example, the compound myosmine was prepared. For the method for synthesizing myosmine, reference is made to a method in the patent publication with No. EP2487172A1. The obtained product was characterized through ¹H NMR. The characterization spectrum is shown in FIG. 1 and the NMR data is as follows: ¹H-NMR (400 MHz, CDCl₃): δ ppm 8.97 (1H, d), 8.64 (1H, dd), 8.17 (1H, dt), 7.29-7.34 (m, 1H), 4.06 (2H), 2.91-2.96 (m, 2H), 2.01-2.09 (m, 2H). It is indicated that myosmine was successfully synthesized. The sample was analyzed and detected through high-performance liquid chromatography. The purity of the sample is 99.3%. The chromatogram is shown in FIG. 2.

### Example 2

In this example, a method for synthesizing (*S*)-nornicotine is provided. The operation method is described below.
3 g of myosmine was added to a 50 mL three-necked round-bottom flask, 10 mL of 0.1 M phosphate buffer was added, and the pH was adjusted to 6.0. 5.5 g of glucose was then added to the reaction flask and stirred to be completely dissolved. 0.2 g of imine reductase IRED103, 0.04 g of glucose dehydrogenase GDH109, and 0.008 g of NADP salt were added to another 50 mL flask and stirred to be completely dissolved. Then, the solution in the second flask was slowly added to the first flask, heated to 25 °C, and stirred at 300 r/min and reacted for 24 h. The solution was filtered, and the filtrate was extracted with chloroform, dried over anhydrous sodium sulfate, and concentrated to obtain 2.2 g of (*S*)-nornicotine.

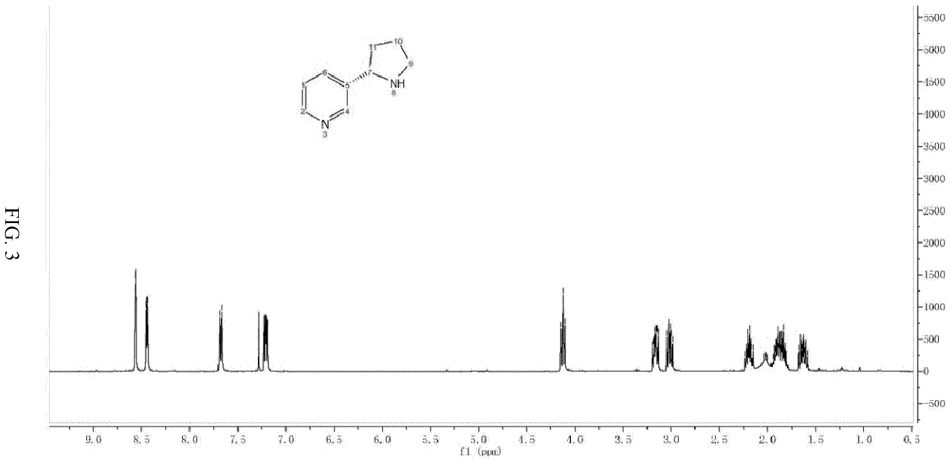

The prepared (*S*)-nornicotine was characterized through ¹H NMR. The characterization spectrum is shown in FIG. 3 and the NMR data is as follows: ¹H-NMR (400 MHz, CDCl₃): δ ppm 8.56 (1H, d), 8.44 (1H, dd), 7.68 (1H, dt), 7.19-7.22 (m,1H), 4.12 (1H,t), 3.13-3.19 (1H, m), 2.98-3.00 (1H, m), 2.15-2.23 (1H, m), 2.02 (1H, s), 1.80-1.93 (2H, m), 1.58-1.67 (1H, m). It is indicated that (*S*)-nornicotine was successfully synthesized.

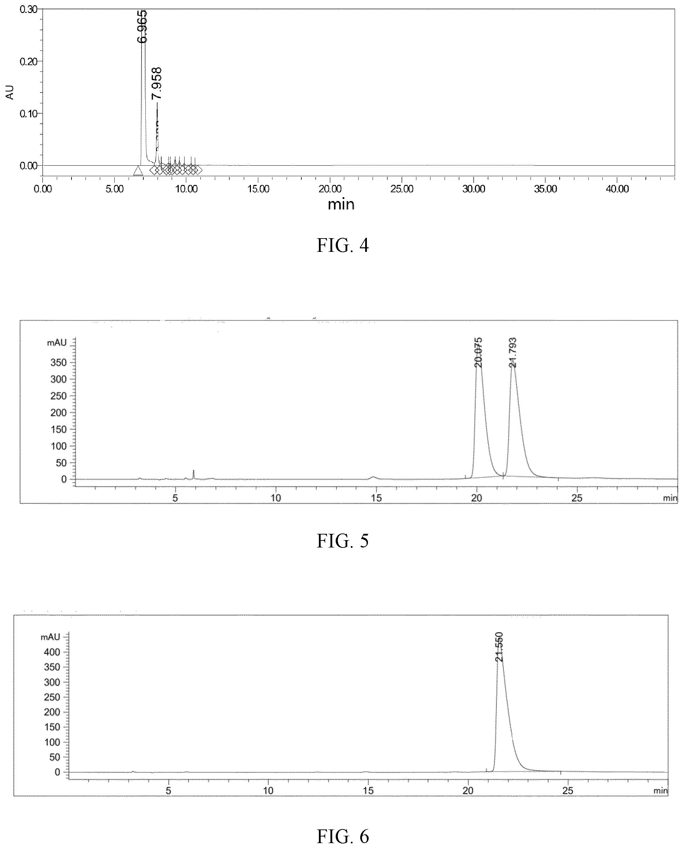

The chemical purity of the prepared (*S*)-nornicotine was detected by a high-performance liquid chromatograph. As shown in FIG. 4, the purity is 93.6%. The optical purity of the sample was detected by a high-performance liquid chromatograph and a chiral chromatography column. FIG. 5 is a chiral separation spectrum of a nornicotine racemate, where the peak appearance time of (*R*)-nornicotine is 20.08 min, the peak appearance time of (*S*)-nornicotine is 21.79 min, and the peak areas of those peaks are 49.9% and 50.1%, respectively; FIG. 6 is a chiral chromatogram of the product, where no (*R*)-nornicotine is detected, and only the peak of (*S*)-nornicotine is detected at 21.6 min, that is, the optical purity of the sample approaches 100%.

### Example 3

In this example, a method for synthesizing (*S*)-nornicotine is provided. The operation method is described below.
3 g of myosmine was added to a 50 mL three-necked round-bottom flask, 15 mL of 0.1 M phosphate buffer was added, and the pH was adjusted to 5.8. 5.5 g of glucose was added to the reaction flask and stirred to be completely dissolved. 0.3 g of imine reductase IRED103, 0.04 g of glucose dehydrogenase GDH102, and 0.01 g of NADP salt were added to another 50 mL flask and stirred to be completely dissolved. Then, the solution in the second flask was slowly added to the first flask, heated to 37 °C, and stirred for 24 h. The solution was filtered, and the filtrate was extracted with chloroform, dried over anhydrous sodium sulfate, and concentrated to obtain 2.4 g of (*S*)-nornicotine.

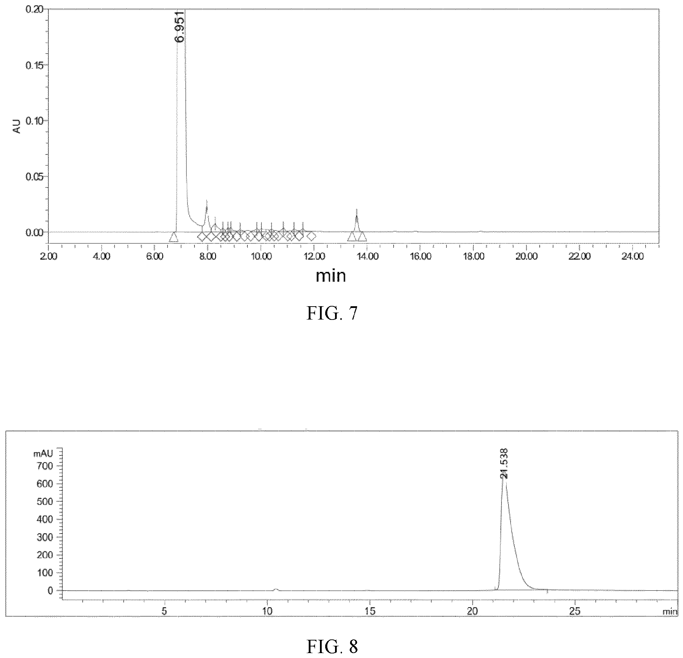

The chemical purity and optical purity of the prepared (*S*)-nornicotine were separately determined by a high-performance liquid chromatograph. The specific operation method is the same as that in Example 2. The chromatogram is shown in FIG. 7, and the chemical purity is calculated to be 97.2%; and the chiral chromatogram is shown in FIG. 8, and the optical purity approaches 100%.

### Example 4

In this example, a method for synthesizing (*S*)-nornicotine is provided. The operation method is described below.
3 g of myosmine was added to a 50 mL three-necked round-bottom flask, 15 mL of 0.1 M phosphate buffer was added, and the pH was adjusted to 5.8. 9 g of glucose was added to the reaction flask and stirred to be completely dissolved. 0.3 g of imine reductase IRED1321110, 0.03 g of glucose dehydrogenase GDH109, and 0.01 g of NADP salt were added to another 50 mL flask and stirred to be completely dissolved. Then, the solution in the second flask was slowly added to the first flask, heated to 37 °C, and stirred for 24 h. The solution was filtered, and the filtrate was extracted with chloroform, dried over anhydrous sodium sulfate, and concentrated to obtain 2.2 g of (*S*)-nornicotine. The optical purity is 99.5%.

### Example 5

In this example, a method for synthesizing (*S*)-nornicotine is provided. The operation method is described below.

3 g of myosmine was added to a 50 mL three-necked round-bottom flask, 15 mL of 0.1 M phosphate buffer was added, and the pH was adjusted to 5.8. 5.5 g of glucose was added to the reaction flask and stirred to be completely dissolved. 0.3 g of imine reductase IRED1321110, 0.06 g of glucose dehydrogenase GDH102, and 0.006 g of NADP salt were added to another 50 mL flask and stirred to be completely dissolved. Then, the solution in the second flask was slowly added to the first flask, heated to 37 °C, and stirred for 24 h. The solution was filtered, and the filtrate was extracted with chloroform, dried over anhydrous sodium sulfate, and concentrated to obtain 2.3 g of (*S*)-nornicotine. The optical purity is 99.5%.

### Example 6

In this example, a method for synthesizing (*S*)-nornicotine is provided. The operation method is described below.
100 g of myosmine was added to a 1000 mL three-necked round-bottom flask, 650 mL of 0.1 M phosphate buffer was added, and the pH was adjusted to 6.2. 180 g of glucose was added to the reaction flask and stirred until the solution was clear. 100 g of a solution of imine reductase IRED103 with a concentration of 10%, 50 g of a solution of glucose dehydrogenase GDH102 with a concentration of 10%, and 5 g of NADP salt were added to a 500 mL flask and stirred until the solution was clear. The solution in the flask was slowly added to the 1000 mL three-necked round-bottom flask, heated to 37 °C, and stirred for 36 h. The solution was filtered, and the filtrate was extracted with chloroform, dried over anhydrous sodium sulfate, and concentrated to obtain 75 g of (*S*)-nornicotine.

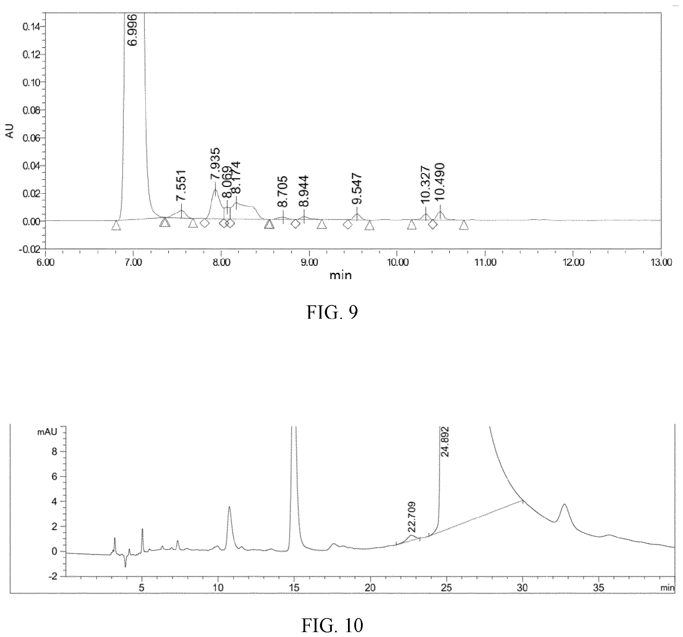

The chemical purity and optical purity of the prepared (*S*)-nornicotine were separately determined by a high-performance liquid chromatograph. The specific operation method is the same as that in Example 2. The chromatogram is shown in FIG. 9, and the chemical purity is calculated to be 95.9%; the chiral chromatogram is shown in FIG. 10, and the optical purity is 99.9%.

### Example 7

In this example, a method for synthesizing (*S*)-nicotine is provided. The operation method is described below.
40 g of (*S*)-nornicotine, 32 g of 37% formaldehyde solution, and 25 g of 85% formic acid solution were added to each of three 500 mL three-necked flasks, heated to 60 °C, and reacted for 24 h. After the reaction was completed, sodium hydroxide was added to adjust the pH to 12. The aqueous phases were extracted with methyl tert-butyl ether, and the extracts were combined, concentrated, and distilled under reduced pressure to obtain 29 g of (*S*)-nicotine as a colorless liquid.

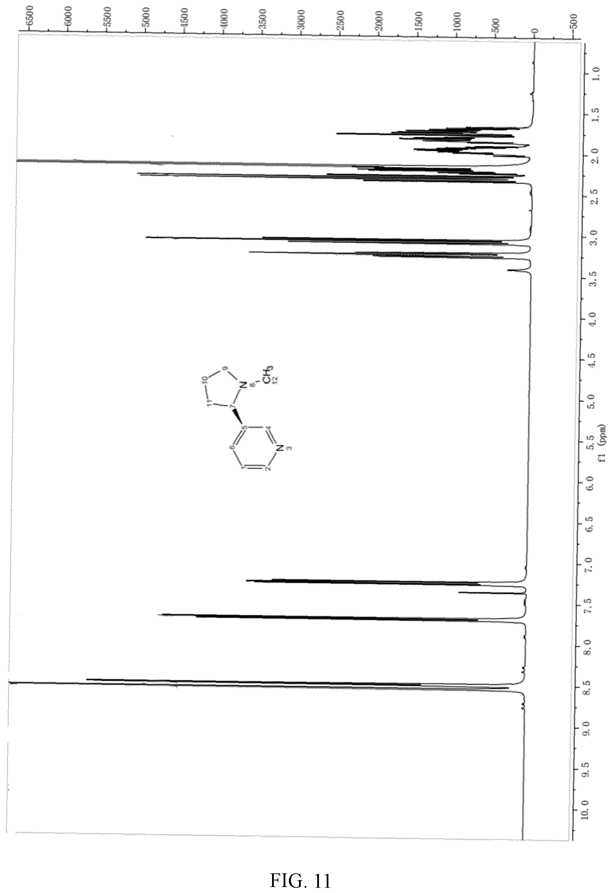

The prepared (*S*)-nicotine was characterized through ¹H NMR. The characterization spectrum is shown in FIG. 11 and the NMR data is as follows: ¹H-NMR (400 MHz, CDCl₃): δ ppm 8.54 (1H, d), 8.50 (1H, dd), 7.70 (1H, dt), 7.24-7.27 (1H, m), 3.22-3.27 (1H, m), 3.08 (1H, t), 2.27-2.34 (1H, m), 2.17-2.24 (1H, m), 2.16 (3H, m), 1.91-2.02 (1H, m), 1.79-1.87 (1H, m), 1.68-1.76 (1H, m). It is indicated that (*S*)-nicotine was successfully synthesized.

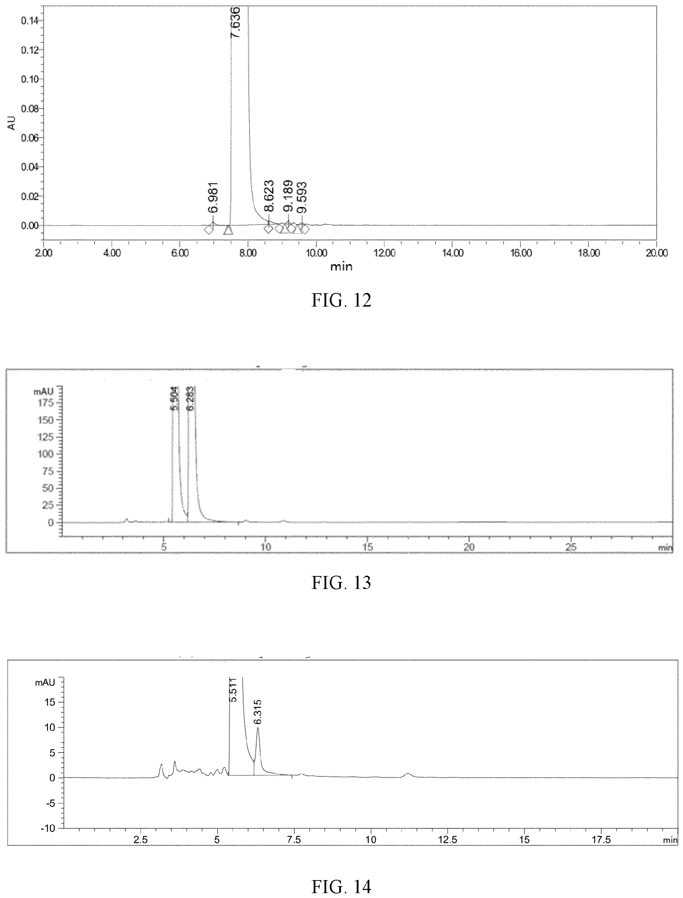

The chemical purity of the prepared (*S*)-nicotine sample was detected by a high-performance liquid chromatograph. As shown in FIG. 12, the detected purity is 99.9%. The optical purity of the sample was detected by a high-performance liquid chromatograph and a chiral chromatography column. FIG. 13 is a chiral chromatogram of a nicotine racemate, where the peak at 5.43 min is (*S*)-nicotine, the peak at 6.17 min is (*R*)-nicotine, and the peak areas of those peaks are 50.1% and 49.9%, respectively; FIG. 14 is a chiral chromatogram of the sample, and it can be seen from the detection chromatogram that the area of the peak at 5.43 min is 99.65%, that is, the purity of (*S*)-nicotine is 99.65%.

### Example 8

In this example, a method for synthesizing (*S*)-nicotine is provided. The operation method is described below.
107 g of (*S*)-nornicotine and 80 g of 37% formaldehyde solution were added to each of three 500 mL three-necked flasks and heated to 75 °C. 60 g of 85% formic acid solution was added dropwise. After the addition, the solution was reacted for 24 h with a temperature maintained. After the reaction was completed, sodium hydroxide was added to adjust the pH to 12. The aqueous phases were extracted with methyl tert-butyl ether, and the extracts were combined, concentrated, and distilled under reduced pressure to obtain 80 g of (*S*)-nicotine as a colorless liquid.

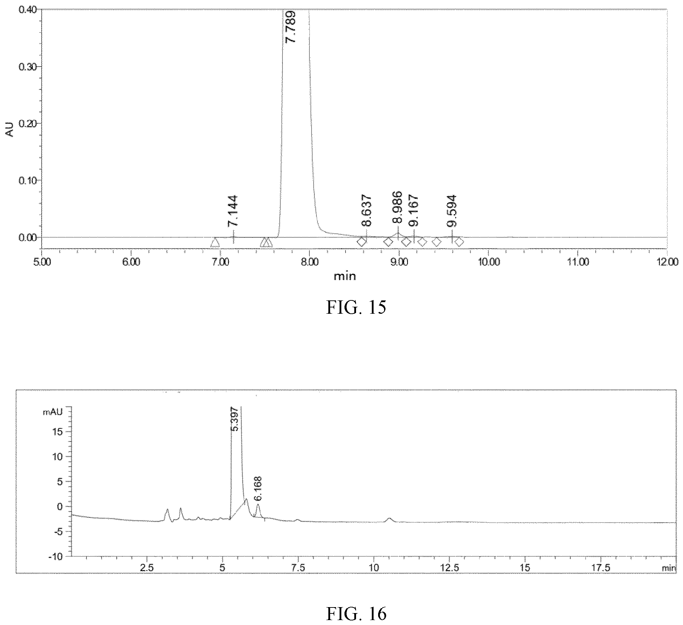

The chemical purity and optical purity of the prepared (*S*)-nicotine were separately determined by a high-performance liquid chromatograph. The specific operations are the same as those in Example 5. The chromatogram is shown in FIG. 15, and the chemical purity is calculated to be 99.7%; and the chiral chromatogram is shown in FIG. 16, and the optical purity is 99.87%.

### Example 9

In this example, a preferred type of imine reductase is discussed. The operation method is described below.

IRED enzyme powder was weighed out and added to 2 mL reaction vials by 10 mg for each. 9.2 mg of glucose, 1 mg of NAD sodium salt, 1 mg of NADP sodium salt, 2 mg of glucose dehydrogenase GDH105, and 900 µL of 0.1 M phosphate buffer (pH 7.0) were added to each vial and shaken sufficiently until the solution was clear. 5 mg of myosmine and 100 µL of DMSO were then added to each vial. The vials were capped tightly, placed in a constant-temperature shaker of 30 °C, and shaken overnight. Samples were analyzed through HPLC for conversion rates and chirality. The results are shown in Table 1.

**Table 1**

| Imine Reductase | Conv. % | e.e% | Imine Reductase | Conv. % | e.e% |
|---|---|---|---|---|---|
| IRED101 | 90.6 | 100 | IRED109 | 36.4 | -89.4 |
| IRED102 | 0 | 0 | IRED110 | 0 | 0 |
| IRED103 | 100 | 100 | IRED111 | 99.8 | -27 |
| IRED104 | 51.1 | 100 | IRED112 | 99.8 | -43.4 |
| IRED105 | 4.1 | 0 | IRED1321104 | 100 | 93.4 |
| IRED106 | 29.2 | 83.8 | IRED1321108 | 100 | 65 |
| IRED107 | 84.7 | 100 | IRED1321110 | 99.6 | 99.9 |
| IRED108 | 99.2 | 38 | IRED1321114 | 100 | 95.4 |

As can be seen from the data in Table 1, IRED103 and IRED1321110 are more preferred types of imine reductase.

### Example 10

In this example, a preferred type of glucose dehydrogenase is discussed. The operation method is described below.
GDH enzyme powder was weighed out and added to 2 mL reaction vials by 2 mg for each. 9.2 mg of glucose, 1 mg of NAD sodium salt, 1 mg of NADP sodium salt, 10 mg of IRED103, and 900 µL of 0.1 M phosphate buffer (pH 7.0) were added to each vial and shaken sufficiently until the solution was clear. 150 mg of myosmine and 100 µL of DMSO were then added to each vial. The vials were capped tightly, placed in a constant-temperature shaker of 30 °C, and shaken overnight. Samples were analyzed through HPLC for conversion rates. The results are shown in Table 2.

**Table 2**

| Glucose Dehydrogenase | Conv. % | Glucose Dehydrogenase | Conv. % |
|---|---|---|---|
| GDH101 | 55 | GDH106 | 20 |
| GDH102 | 96.6 | GDH107 | 21 |
| GDH103 | 2.7 | GDH108 | 0 |
| GDH104 | 16 | GDH109 | 100 |
| GDH105 | 27 | | |

As can be seen from the data in Table 2, GDH109 is a more preferred type of glucose dehydrogenase.

### Example 11

In this example, a preferred type of coenzyme is discussed. The operation method is described below.
1 mg of NAD sodium salt and 1 mg of NADP sodium salt were weighed and added to two 2 mL reaction vials, respectively. 9.2 mg of glucose, 2 mg of GDH109 enzyme powder, 10 mg of IRED103, and 900 µL of 0.1 M phosphate buffer (pH 7.0) were added to each vial and shaken sufficiently until the solution was clear. 150 mg of myosmine and 100 µL of DMSO were then added to each vial. The vials were capped tightly, placed in a constant-temperature shaker of 30 °C, and shaken overnight. Samples were analyzed through HPLC for conversion rates. The results are shown in Table 3.

**Table 3**

| Coenzyme | Conv.% |
|---|---|
| NAD sodium salt | 25.9 |
| NADP sodium salt | 100 |

As can be seen from the data in Table 3, the NADP sodium salt is a more preferred type of coenzyme.

The applicant has stated that although the method for synthesizing (*S*)-nicotine in the present application is described through the examples described above, the present application is not limited to the examples described above, which means that the implementation of the present application does not necessarily depend on the examples described above. It should be apparent to those skilled in the art that any improvements made to the present application, equivalent replacements of raw materials of the product, additions of adjuvant ingredients, selections of specific methods, etc. in the present application all fall within the protection scope and the disclosure scope of the present application.

Although the preferred embodiments of the present application are described above in detail, the present application is not limited to details in the embodiments described above, and various simple modifications can be made to the technical solutions of the present application without departing from the technical concept of the present application. These simple modifications are all within the protection scope of the present application.

Additionally, it is to be noted that if not in collision, the specific technical features described in the embodiments described above may be combined in any suitable manner. In order to avoid unnecessary repetition, various possible combination manners are no longer described in the present application.

## Claims

1. A method for synthesizing (*S*)-nicotine, comprising
under the condition of a coenzyme circulation system, with a coenzyme as a hydrogen donor and an imine reductase as a catalyst, subjecting myosmine to catalytic reduction to obtain (*S*)-nornicotine and subjecting (*S*)-nornicotine to methylation to obtain the (*S*)-nicotine.

2. The method for synthesizing (*S*)-nicotine of claim 1, wherein the coenzyme circulation system comprises a coenzyme, glucose, and a glucose dehydrogenase.

3. The method for synthesizing (*S*)-nicotine of claim 1 or 2, wherein the coenzyme comprises a NADP salt and/or a NAD salt, preferably an NADP salt.

4. The method for synthesizing (*S*)-nicotine of claim 2 or 3, wherein the glucose dehydrogenase comprises any one or a combination of at least two of GDH101 to GDH109, preferably GDH109.

5. The method for synthesizing (*S*)-nicotine of any one of claims 1 to 4, wherein the imine reductase comprises any one or a combination of at least two of IRED101 to IRED112, IRED1321104, IRED1321108, IRED1321110 or IRED1321114, preferably IRED103 or IRED1321110.

6. The method for synthesizing (*S*)-nicotine of any one of claims 1 to 5, wherein the catalytic reduction is performed at a temperature of 15-45 °C, preferably 25-30 °C;
preferably, the catalytic reduction is performed for 8-72 h;
preferably, the catalytic reduction is performed under the condition of stirring at a rotational speed of 150-400 r/min.

7. The method for synthesizing (*S*)-nicotine of any one of claims 1 to 6, wherein the catalytic reduction is performed in a buffer, wherein the buffer comprises a phosphate buffer, a tris(hydroxymethyl)methylamine-hydrochloride buffer, or a triethanolamine-hydrochloride buffer;
preferably, the buffer has a pH of 5.0-9.0, preferably 5.8-6.5.

8. The method for synthesizing (*S*)-nicotine of any one of claims 1 to 7, wherein the methylation comprises mixing and reacting (*S*)-nornicotine with formaldehyde and formic acid to obtain the (*S*)-nicotine.

9. The method for synthesizing (*S*)-nicotine of any one of claims 1 to 8, wherein the methylation is performed at a temperature of 55-80 °C;
preferably, the methylation is performed for 8-48 h.

10. The method for synthesizing (*S*)-nicotine of any one of claims 1 to 9, comprising
mixing myosmine, a coenzyme, glucose, a glucose dehydrogenase, a buffer and an imine reductase and reacting the mixture at 15-45 °C for 8-72 h to obtain (*S*)-nornicotine, wherein a mass ratio of myosmine, the coenzyme, and the imine reductase is 1:(0.001-0.05):(0.05-0.1); and then mixing (*S*)-nornicotine with formaldehyde and formic acid and reacting the mixture at 55-80 °C for 8-48 h to obtain the (*S*)-nicotine.
